# EUROPEAN PATENT SPECIFICATION

(11) **EP 3 162 285 B1**
(45) Date of publication and mention of the grant of the patent: **08.12.2021**
(21) Application number: 15191622.8
(22) Date of filing: 27.10.2015
(51) Int. Cl.: A61B 5/024, A61B 5/00, G02B 6/08

(54) **A SENSOR ELEMENT**
SENSORELEMENT
ÉLÉMENT CAPTEUR

(43) Date of publication of application: 03.05.2017
(73) Proprietor: Nokia Technologies Oy, 02610 Espoo (FI)
(72) Inventor: LASAROV, Harri, 02280 Espoo (FI); BLOMQVIST, Kim, 02650 Espoo (FI); SALO, Antti, 08500 Lohja as. (FI)
(74) Representative: Espatent Oy

(56) References cited:
- EP-A2- 2 781 187
- US-A1- 2004 187 553
- US-A1- 2008 232 751
- US-A1- 2010 010 340
- US-A1- 2013 301 294
- US-A1- 2015 112 207

## Description

### TECHNICAL FIELD

The present application generally relates to sensor elements and particularly to optical sensor elements.

### BACKGROUND

This section illustrates useful background information without admission of any technique described herein representative of the state of the art.

Various metering devices and sensors that measure physiological conditions of users such as pulse sensors have become common for people to measure their own heart rate, movements or other parameters. The measurements can be performed using a chest strap that is worn under clothes or using a wrist worn watch-like sensor device. The sensors measure physiological conditions of a user and produce sensor signals corresponding to a property of the skin of the user or underlying matter (capillaries and veins, for example).

Pulse or heart rate can be monitored for example optically using a photoplethymography (PPG) sensor. Optical sensors such as PPG sensors need to be sealed with optically transparent method, e.g. with glass window, to protect against dust and moisture.

EP2781187 discloses a device according to the preamble of claim 1 including light shielding units around and in between a light emitter and a light detector.

US 2008 / 0232751 A1 discloses a medical imaging apparatus with light emitters and detectors comprising optical fibre faceplates.

### SUMMARY

Various aspects of examples of the invention are set out in the claims. The embodiments and features, if any, described in this specification that do not fall under the scope of the independent claims are to be interpreted as examples useful for understanding various embodiments of the invention.

According to a first example aspect of the present invention, there is provided a sensor element according to claim 1.

According to a second example aspect of the present invention, there is provided an apparatus comprising a cover and a sensor element of the first aspect, wherein at least part of the cover and the medium in-between the optical fibers of the window element of the sensor element have the same color to camouflage contents of the sensor element.

According to a third example aspect of the present invention, there is provided a method according to claim 13.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 is a simplified illustration of an example optical heart rate measurement;
Fig. 2 is a simplified cross-sectional illustration of an optical sensor and internal light pollution in the sensor;
Fig. 3 is a simplified cross-sectional illustration of an optical sensor according to an embodiment of the invention;
Figs. 4A-4C show simplified top-views of window elements of some example embodiments of the invention;
Fig. 5 illustrates behavior of a window element;
Fig. 6 shows simplified top-view illustrations of optical sensors;
Fig. 7 is a simplified cross-sectional illustration of an optical sensor according to an embodiment of the invention;
Fig. 8 is a simplified cross-sectional illustration of an optical sensor according to an embodiment of the invention;
Fig. 9 is a simplified profile view of a window element according to an embodiment of the invention;
Fig. 10 shows example measurement results; and
Fig. 11 shows a flow chart of a process of an example embodiment.

### DETAILED DESCRIPTION OF THE DRAWINGS

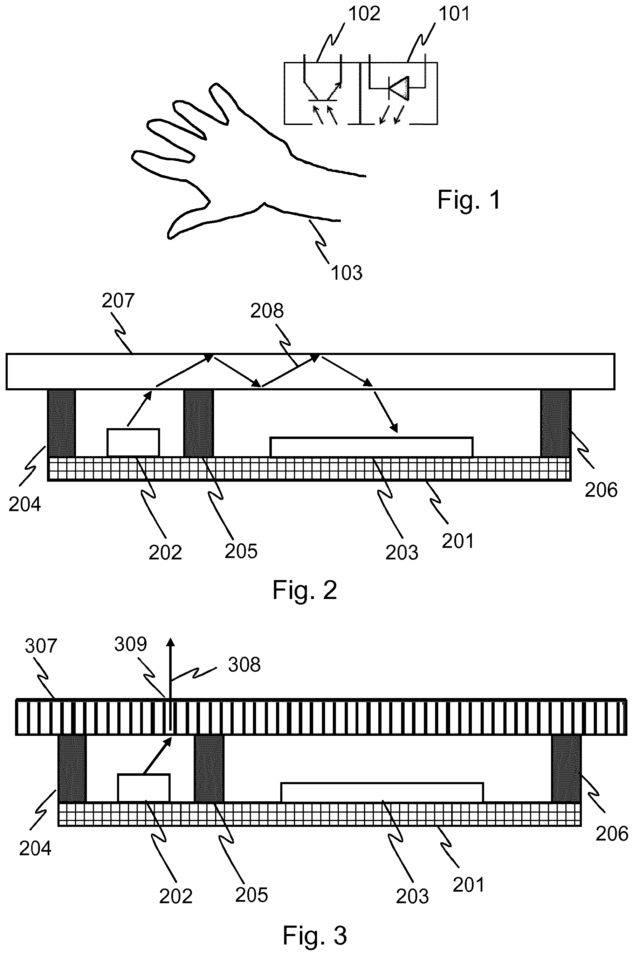

Example embodiments of the present invention and its potential advantages are understood by referring to Figs. 1 through 11 of the drawings. In this document, like reference signs denote like parts or steps.

In the following, various example embodiments are discussed in connection with optical heart rate sensors. Various embodiments are however not necessarily limited to optical heart rate sensor only. Instead the example embodiments can be used in monitoring some other physiological condition and/or in some other type of optical sensors, too. Physiological conditions or physiological measurement results referred to herein may include one or more of the following: heart rate, respiration rate, blood pressure, oxygen saturation level, and glucose level. Also some other optical measurements or optical sensing may apply, such as camera sensors and flash lights or infrared (IR) sensors.

Heart rate can be monitored optically by measuring variations in blood volume with a photoplethymography (PPG) sensor. Fig. 1 is a simplified illustration of an example optical heart rate measurement. Fig. 1 shows a (reflective type) PPG sensor that comprises a LED (light emitting diode) 101, a light source, and a photo transistor 102, a light detector. Also a photo diode (PD) may be used as the light detector. The LED (optical emitter, light source) 101 emits light and the light detector 102 receives light rays reflected from a wrist 103 of a user. The sensor produces sensor signals based on the light detected by the light detector 102. A reflective type sensor is shown here only as an example of an optical sensor. It is understood that embodiments of the invention are suited for other (optical) sensors as well.

Optical sensors such as PPG sensors need to be sealed with optically transparent method, e.g. with glass window, to protect against dust and moisture. Cross talk of light rays through such glass window may cause internal light pollution (ILP) problem as light rays from the light source may travel directly to the light detector.

Fig. 2 is a simplified cross-sectional illustration of an optical sensor and internal light pollution in the sensor. The optical sensor comprises a printed circuit board (PCB) 201, also known as a printed wiring board (PWB), a light emitting diode (LED) 202 attached to the PCB 201, a photodiode 203 attached to the PCB 201 and walls 204-206 surrounding the LED 202 and the photodiode 203. Further the optical sensor comprises a glass window 207 that covers and protects the LED 202 and the photodiode 203. Arrows 208 illustrate how some of the light rays emitted by the LED 202 travel directly to the photodiode without exiting the optical sensor due to refraction in the glass window.

In various example embodiments of the invention there is provided a sensor element with a window element made of a plurality of adjacent optical fibers (a bunch or a bundle of optical fibers). The window element covers a light source and a light detector of the sensor element. In this way internal light pollution may be reduced. In an embodiment the window element is a fiber optic faceplate. In an embodiment the window element comprises one or more fiber optic tapers.

In an embodiment the adjacent optical fibers are aligned. In an embodiment the adjacent optical fibers form a structure that comprises at least one flat surface (e.g. a plate like structure or a disk like structure or other flat structure). Thickness of the structure formed by the optical fibers may be uniform or there may be variations in the thickness. It may be defined that the optical fibers of the window element point to (and convey light to) more or less same direction (substantially same direction). One may define that the optical fibers form a structure with a first and a second opposite surface and the optical fibers convey light from the first surface to the second surface or vice versa. The optical fibers may be parallel, but this not mandatory. In some embodiments the optical fibers may point to different directions. Characteristics of the optical fibers may be identical or there may be optical fibers with different characteristics. For example, size/form of the optical fibers may be the same or there may be optical fibers of different sizes/forms.

In an embodiment the window element comprises suitable medium / material / substance between the optical fibers / between core material of the optical fibers. This material may be referred to as intervening material or filling material or interstitial material that fills or forms space/interstices between the optical fibers or separates the optical fibers / core material of the optical fibers from each other. In an embodiment cladding material surrounding the core material of the optical fibers forms the medium in-between the optical fibers of the window element.

Fig. 3 is a simplified cross-sectional illustration of an optical sensor according to an embodiment of the invention. The optical sensor comprises a printed circuit board (PCB) 201, a light emitting diode (LED) 202 attached to the PCB 201, a photodiode 203 attached to the PCB 201, also known as a printed wiring board (PWB), and walls 204-206 surrounding the LED 202 and the photodiode 203. Further the optical sensor comprises a window element 307 that covers and protects the LED 202 and the photodiode 203. The window element comprises a plurality of adjacent optical fibers 309.

Arrows 308 illustrate how light rays emitted by the LED 202 exit the sensor element without cross talk thereby reducing internal light pollution in the sensor element.

Fig 3 shows an empty space between the LED 202/photodiode 203 and the window element 307, but this is not mandatory feature. In an alternative implementation the window element 307 may be in direct contact with the LED 202 and/or the photodiode 203. In an embodiment the empty space between the LED 202/photodiode 203 and the window element 307 may be filled with suitable optical material, such as one of the following: epoxy, silicone and acrylic type material.

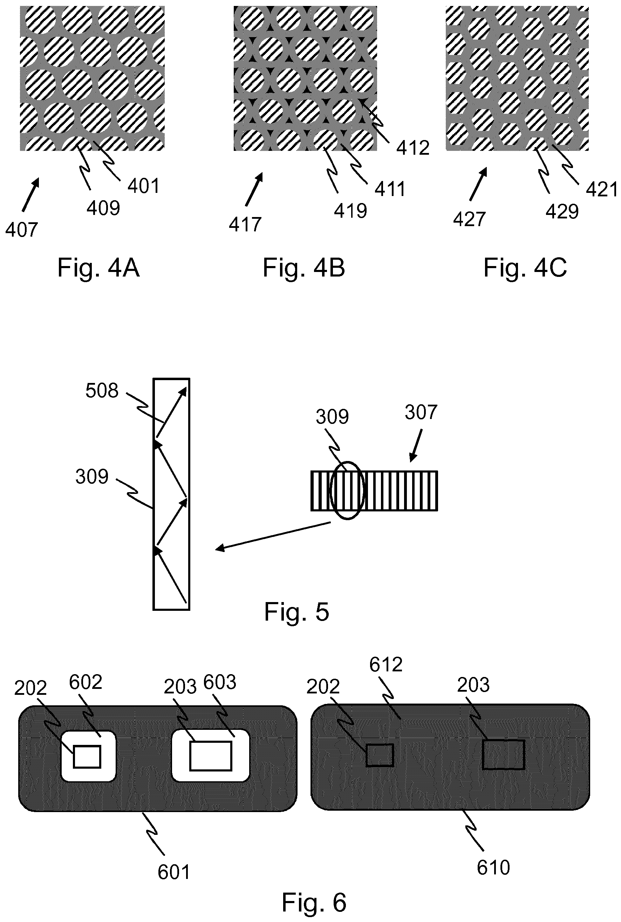

The window element of various embodiments of the invention, e.g. the window element 307 of Fig 3, may be for example a fiber optic faceplate (FOFP). The window element may be manufactured for example by slicing fused boules of optical fibers. Thickness of the window element may be for example 0,5 - 1,5 mm, but also thicker or thinner windows can be used. Figs. 4A-4C show simplified top-views of window elements of some example embodiments of the invention (e.g. a fiber optic faceplate).

Fig. 4A shows a window element 407 comprising a plurality of optical fibers 409 and medium 401 between the optical fibers 409. Fig. 4B shows a window element 417 comprising a plurality of optical fibers 419 and cladding 411 surrounding core material of the optical fibers 419. The cladding 411 forms the medium in-between the optical fibers according to various embodiments of the invention between the optical fibers 419. The window element 417 may comprise further material in openings/space 412 between the cladding 411. Alternatively these openings 412 may be empty. Fig. 4C shows a window element 427 comprising a plurality of optical fibers 429 with hexagonal cross section and medium 421 between the optical fibers 429. Cladding of the optical fibers 429 may form the medium 421 between the optical fibers 429. It is understood that Figs. 4A-4C show only some examples and also other options or some combination of the shown examples are possible.

Fig. 5 illustrates behavior of a window element 307. Fig. 5 shows a magnification of one optical fiber 309 of the window element 307. Arrows 508 show how light rays go through the optical fiber 309 without refraction to side-directions (total internal reflection) thereby eliminating or reducing cross talk and thus reducing internal light pollution.

In an embodiment the medium in-between the optical fibers (e.g. the cladding material of the window element) is tinted to have a predefined color. In an embodiment the medium in-between the optical fibers is non-transparent in certain wavelength range. In an embodiment the medium in-between the optical fibers is non-transparent in visible light (electromagnetic spectrum that is visible to human eye) but may be transparent in some other range of wavelengths, too. Visible light wavelength range may refer to wavelengths around 400-700 nm. In an embodiment the window element is made of glass or plastic. In practice the optical fibers forming the window element may be made of glass or plastic. In an embodiment the medium in-between the optical fibers may be for example glass or plastic.

By tinting the medium in-between the optical fibers to have the same color as surrounding apparatus cover it is possible to camouflage sensor components / to fade the window area so that the window area does not stand out from the apparatus cover.

Fig. 6 shows simplified top-view illustrations of optical sensors. Optical sensor 601 comprises a conventional protective window with colored back printing. The back printing has openings 602 and 603 for LED 202 and photodiode 203 of the optical sensor. The LED 202 and the photodiode 203 are visible to the user through the openings 602 and 603. This may not be desired visual appearance.

Optical sensor 610 comprises a window element according to an embodiment of the invention. The medium in-between the optical fibers of the window element is tinted and thereby the LED 202 and the photodiode 203 underneath the window element are nearly invisible. In this way better visual appearance is obtained. In an embodiment also the core material of the optical fibers of the window element is tinted whereby even better hiding of the contents of the optical sensor is obtained.

There are also other alternatives to the implementation shown in Fig 3. Some alternatives are discussed in the following. It is to be understood that features of different alternatives may be combined with features of other alternatives.

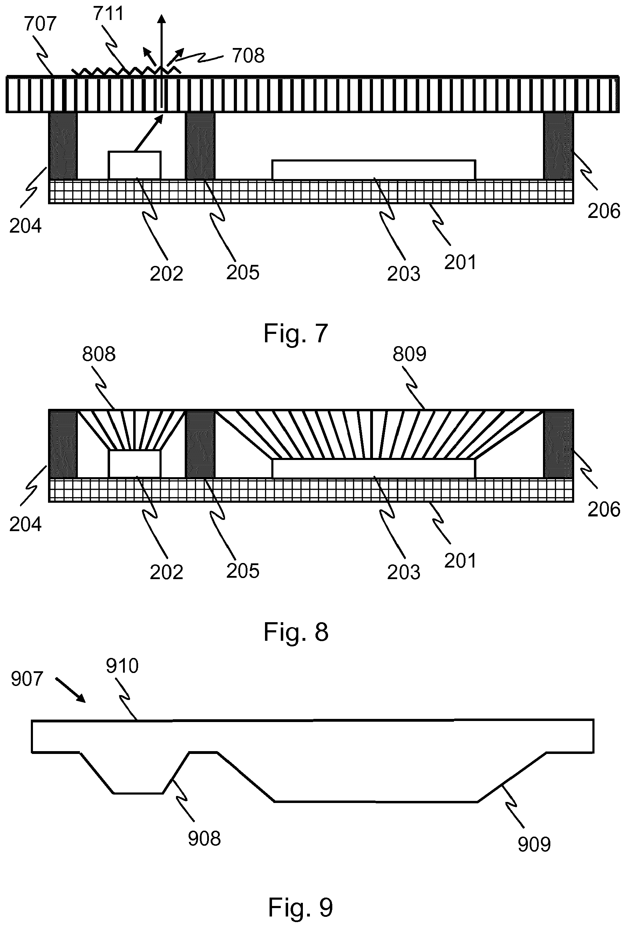

Fig. 7 is a simplified cross-sectional illustration of an optical sensor according to an embodiment of the invention. The optical sensor comprises a printed circuit board (PCB) 201, a light emitting diode (LED) 202 attached to the PCB 201, a photodiode 203 attached to the PCB 201 and walls 204-206 surrounding the LED 202 and the photodiode 203. Further the optical sensor comprises a window element 707 that covers and protects the LED 202 and the photodiode 203. The window element comprises a plurality of adjacent optical fibers and a roughened surface 711.

Arrows 708 illustrate how light rays emitted by the LED 202 exit the sensor element are effectively spread to the exterior of the sensor element as there exist number of light scattering surface areas in the roughened surface.

In the example shown in Fig 7, there is roughened surface on top of the LED 202, i.e. in the area where light is intended to exit the sensor element. It is however possible that also other areas of the surface are roughened or that only part of the area covering the LED 202 is roughened. The surface of the window element 707 may be roughened for example by chemical etching.

In an embodiment diffractive optics or diffractive structure are used instead of the roughened surface of Fig 7 to direct and spread light entering or exiting the window element according to embodiments of the invention. A diffractive structure pattern on surfaces enables to direct light primarily via diffraction or may also be configured to guide light via refraction. The diffractive structure may be used on outside facing window surface or inside facing window surface or both. The diffractive structure may, for example, be a diffractive optical element, a diffraction grating, a periodic structure/pattern or a series of diffraction lines/slits/grooves.

Fig. 8 is a simplified cross-sectional illustration of an optical sensor according to an embodiment of the invention. The optical sensor comprises a printed circuit board (PCB) 201, a light emitting diode (LED) 202 attached to the PCB 201, a photodiode 203 attached to the PCB 201 and walls 204-206 surrounding the LED 202 and the photodiode 203. Further the optical sensor comprises two window elements 808 and 809 that cover and protect the LED 202 and the photodiode 203. The window elements 808 and 809 comprise a plurality of adjacent and tapered optical fibers. The window element 808 is configured to spread light emitted by the LED 202 to the exterior of the sensor element and the window element 809 is configured to focus incoming light to the photodiode 203. In an embodiment the window elements 808 and 809 are fiber optic tapers.

In the example shown in Fig. 8, the window elements 808 and 809 touch / are in contact with the LED 202 and the photodiode 203, i.e. there is no gap between these. In an alternative embodiment there may be a gap between the window element 808 and/or 809 and the LED 202 and/or the photodiode 203. This gap may an empty gap or the gap may be filled with suitable optical material, such as one of the following: epoxy, silicone and acrylic type material.

In the example of Fig. 8 two separate tapered window elements are used. Alternatively the tapers may be formed into one element. Fig. 9 is a simplified profile view of a window element 907 according to an embodiment of the invention. The shown example window element 907 comprises two tapered sections 908 and 909 and a flat section 910 connecting the tapered sections 908 and 909. In an example embodiment tapers 908 and 909 are fused together. In an example embodiment CNC (computer numerical control) machines are used to generate the desired profile of the window element. With CNC machines it may be possible to obtain different shapes quite freely.

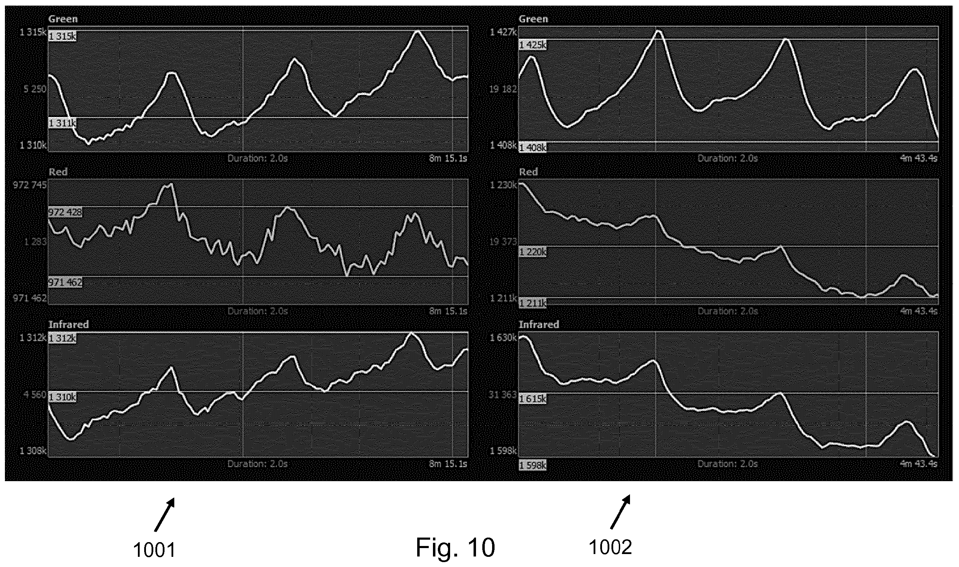

Fig. 10 shows example measurement results obtained in preliminary test environment for green, red and infra-red wavelengths. The chart shows measurement results 1001 with a glass window having an example thickness of 0,4 mm and measurement results 1002 with a fiber optic faceplate having an example thickness of 1,6 mm. From the test results it can be seen that higher gain and amplitude are obtained with the FOFP in comparison to the glass window. Additionally, preliminary dark room tests showed that there was over 40% decrease in internal light pollution with the FOFP in comparison to the glass window. It is to be noted that these are results obtained in simple test environment and that results in an actual implementation may differ from these.

The sensor element of various embodiments of the invention may be part of an electronic apparatus such as a handheld apparatus or a user wearable apparatus. In an embodiment the medium in-between the optical fibers of the window element and possibly also the core material of the optical fibers in the window element are tinted to have the same color with the cover or casing of the electronic apparatus to hide the contents of the sensor element and thereby to improve visual appearance. The handheld apparatus may be for example a communication apparatus such as a mobile phone or the like. The user wearable apparatus may be an apparatus that can be fitted around a body part (e.g. wrist or ankle) of a user using a strap. The strap may be made of suitable flexible or bendable material, such as plastic, fabric, and leather. In an example embodiment, the strap and the body are integrally formed of one piece of material. The material can comprise or consist of any of the following: plastics, metals, nano-fibers, carbon fiber, leather, fabric and glass. The user wearable apparatus may be a device that is configured to be integrated into a garment of a user. The user wearable apparatus may be attached or integrated for example to a belt, a sock, a shoe, a sleeve or a collar of a shirt or pullover, and/or a waistband of trousers or skirt. The user wearable apparatus may be detachable from the garment. The user wearable apparatus may be shaped like a watch and it may be configured to display time or other useful information to the user.

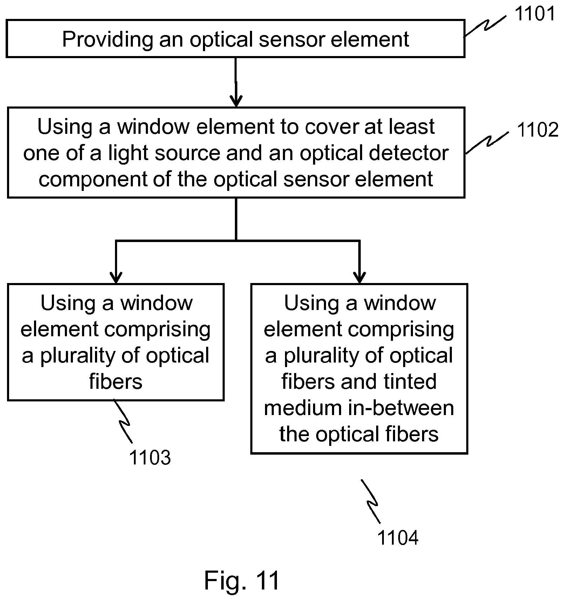

Fig. 11 shows a flow chart of a method of an example embodiment. The process comprises:
1101: An optical sensor element is provided. The optical sensor element comprises a light source and an optical detector component.
1102: A window element is used to cover the light source and the optical detector component.
1103: In an embodiment the window element that is used comprises a plurality of adjacent optical fibers.
1104: In an alternative embodiment the window element that is used comprises a plurality of adjacent optical fibers and tinted medium in-between the optical fibers. The medium in-between the optical fibers is tinted to have desired color or transparency/non-transparency.

It is to be noted that implementation details of window elements according to embodiments of the invention may vary. For example, diameter of the optical fibers in the window element may vary. In an example there are fibers with larger diameter in the middle of the window element and fibers with smaller diameter towards the edges of the window element. In yet another example different tints may be used in different areas of the window element. For example certain area may have black tint and some other area may have red tint. In this way wide range of visual effects can be obtained.

In the shown examples both the light source and the detector element are covered with the window element made of optical fibers. In an alternative embodiment only the light source or the detector element are covered with such window element and the other element may be covered with conventional glass window for example. Such solution may suffice for obtaining the effect of reducing internal light pollution.

It is noted that in shown examples, only one light source and one light detector are shown. It is however understood that there may exist more than one light source and/or more than one light detectors in one sensor element.

Without in any way limiting the scope, interpretation, or application of the claims appearing below, a technical effect of one or more of the example embodiments disclosed herein is an improved sensor element. Another technical effect of one or more of the example embodiments disclosed herein is reduced internal light pollution since direct light from the light source and/or reflections through the window to the light detector can be reduced. Another technical effect of one or more of the example embodiments disclosed herein is a camouflage effect whereby contents of a sensor element (e.g. light source and/or light detector elements) may be at least partially hidden.

If desired, the different functions discussed herein may be performed in a different order and/or concurrently with each other. Furthermore, if desired, one or more of the before-described functions may be optional or may be combined.

It is also noted herein that while the foregoing describes example embodiments of the invention, these descriptions should not be viewed in a limiting sense. Rather, there are several variations and modifications, which may be made without departing from the scope of the present invention as defined in the appended claims.

## Claims

1. A sensor element, wherein the sensor element is an optical sensor configured to sense physiological conditions, the sensor element comprising:
a light source (202) configured to emit light to the exterior of the sensor element, and
a light detector (203) configured to detect light that enters the sensor element, **characterized in that** the sensor element comprises
walls (204-206) surrounding the light source (202) and the light detector (203), and
a window element (307, 707, 808, 809, 907) in contact with the walls (204-206), wherein the window element covers and protects the light source (202) and the light detector (203), wherein the window element comprises a plurality of adjacent optical fibers (309, 409, 419, 429).

2. The sensor element of claim 1, wherein said optical fibers (309) are aligned.

3. The sensor element of claim 1 or 2, wherein said window element comprises a fiber optic faceplate.

4. The sensor element of claim 1 or 2, wherein said window element comprises a fiber optic taper.

5. The sensor element of any preceding claim, wherein the optical fibers (309, 409, 419, 429) of the window element (307, 707) are configured to guide light from the light source (202) to the exterior of the sensor element or from the exterior of the sensor element to the light detector (203).

6. The sensor element of any preceding claim, wherein the window element comprises a medium (401, 411, 412, 421) in-between the optical fibers.

7. The sensor element of claim 6, wherein the medium in-between the optical fibers comprises cladding material surrounding core material of the optical fibers.

8. The sensor element of claim 6 or 7, wherein the medium in-between the optical fibers is tinted material.

9. The sensor element of any one of claims 5-8, wherein the medium in-between the optical fibers is non-transparent in certain wavelength range.

10. The sensor element of any one of claims 6-9, wherein the medium in-between the optical fibers is non-transparent in visible light.

11. The sensor element of any one of claims 6-10, wherein the medium in-between the optical fibers and core material of the optical fibers are tinted.

12. An apparatus comprising a cover and a sensor element of any one of claims 6-11, wherein at least part of the cover and the medium in-between the optical fibers of the window element of the sensor element have the same color to camouflage contents of the sensor element.

13. A method comprising:
providing (1101) a sensor element comprising a light source (202) configured to emit light to the exterior of the sensor element and a light detector (203) configured to detect light that enters the sensor element, wherein the sensor element is an optical sensor configured to sense physiological conditions; **characterized by**
providing walls (204-206) to surround the light source (202) and the light detector (203), and using (1102) a window element (307, 707, 808, 809, 907) in contact with the walls to cover and protect the light source (202) and the light detector (203), wherein the window element comprises a plurality of adjacent optical fibers (309, 409, 419, 429).

14. A method according to claim 13, wherein the window element comprises tinted medium in-between the optical fibers (309, 409, 419, 429).

## Patentansprüche

1. Sensorelement, wobei das Sensorelement ein optischer Sensor ist, der dazu ausgelegt ist, physiologische Zustände zu erfassen, wobei das Sensorelement Folgendes umfasst:
eine Lichtquelle (202), die dazu ausgelegt ist, Licht zur Außenseite des Sensorelements zu emittieren, und
einen Lichtdetektor (203), der dazu ausgelegt ist, Licht, das in das Sensorelement eintritt, zu detektieren, **dadurch gekennzeichnet, dass** das Sensorelement Folgendes umfasst
Wände (204-206), die die Lichtquelle (202) und den Lichtdetektor (203) umgeben, und
ein Fensterelement (307, 707, 808, 809, 907) in Kontakt mit den Wänden (204-206), wobei das Fensterelement die Lichtquelle (202) und den Lichtdetektor (203) abdeckt und schützt, wobei das Fensterelement eine Vielzahl von benachbarten Lichtwellenleitern (309, 409, 419, 429) umfasst.

2. Sensorelement nach Anspruch 1, wobei die Lichtwellenleiter (309) ausgerichtet sind.

3. Sensorelement nach Anspruch 1 oder 2, wobei das Fensterelement eine Lichtwellenleiterfrontplatte umfasst.

4. Sensorelement nach Anspruch 1 oder 2, wobei das Fensterelement eine Lichtwellenleiterabschrägung umfasst.

5. Sensorelement nach einem der vorhergehenden Ansprüche, wobei die Lichtwellenleiter (309, 409, 419, 429) des Fensterelements (307, 707) dazu ausgelegt sind, Licht von der Lichtquelle (202) zur Außenseite des Sensorelements oder von der Außenseite des Sensorelements zum Lichtdetektor (203) zu leiten.

6. Sensorelement nach einem der vorhergehenden Ansprüche, wobei das Fensterelement zwischen den Lichtwellenleitern ein Medium (401, 411, 412, 421) umfasst.

7. Sensorelement nach Anspruch 6, wobei das Medium zwischen den Lichtwellenleitern ein Ummantelungsmaterial umfasst, das das Kernmaterial der Lichtwellenleiter umgibt.

8. Sensorelement nach Anspruch 6 oder 7, wobei das Medium zwischen den Lichtwellenleitern getöntes Material ist.

9. Sensorelement nach einem der Ansprüche 5 bis 8, wobei das Medium zwischen den Lichtwellenleitern in einem bestimmten Wellenlängenbereich nicht transparent ist.

10. Sensorelement nach einem der Ansprüche 6 bis 9, wobei das Medium zwischen den Lichtwellenleitern in sichtbarem Licht nicht transparent ist.

11. Sensorelement nach einem der Ansprüche 6 bis 10, wobei das Medium zwischen den Lichtwellenleitern und das Kernmaterial der Lichtwellenleiter getönt ist.

12. Vorrichtung, die eine Abdeckung und ein Sensorelement nach einem der Ansprüche 6 bis 11 umfasst, wobei mindestens ein Teil der Abdeckung und das Medium zwischen den Lichtwellenleitern des Fensterelements des Sensorelements dieselbe Farbe aufweisen, um den Inhalt des Sensorelements zu verbergen.

13. Verfahren, das Folgendes umfasst:
Bereitstellen (1101) eines Sensorelements, das eine Lichtquelle (202), die dazu ausgelegt ist, Licht zur Außenseite des Sensorelements zu emittieren, und einen Lichtdetektor (203), der dazu ausgelegt ist, Licht, das in das Sensorelement eintritt, zu detektieren, umfasst, wobei das Sensorelement ein optischer Sensor ist, der dazu ausgelegt ist, physiologische Zustände zu erfassen; **gekennzeichnet durch**
Bereitstellen von Wänden (204-206), um die Lichtquelle (202) und den Lichtdetektor (203) zu umgeben, und Verwenden (1102) eines Fensterelements (307, 707, 808, 809, 907) in Kontakt mit den Wänden, um die Lichtquelle (202) und den Lichtdetektor (203) abzudecken und zu schützen, wobei das Fensterelement eine Vielzahl von benachbarten Lichtwellenleitern (309, 409, 419, 429) umfasst.

14. Verfahren gemäß Anspruch 13, wobei das Fensterelement zwischen den Lichtwellenleitern (309, 409, 419, 429) ein getöntes Medium umfasst.

## Revendications

1. Élément capteur, dans lequel l'élément capteur est un capteur optique configuré pour détecter des conditions physiologiques, dans lequel l'élément capteur comprend :
une source de lumière (202) configurée pour émettre de la lumière vers l'extérieur de l'élément capteur, et
un détecteur de lumière (203) configuré pour détecter la lumière qui pénètre dans l'élément capteur, **caractérisé en ce que** l'élément capteur comprend
des parois (204-206) qui entourent la source de lumière (202) et le détecteur de lumière (203), et
un élément de fenêtre (307, 707, 808, 809, 907) en contact avec les parois (204-206), dans lequel l'élément de fenêtre recouvre et protège la source de lumière (202) et le détecteur de lumière (203), dans lequel l'élément de fenêtre comprend une pluralité de fibres optiques adjacentes (309, 409, 419, 429).

2. Élément capteur selon la revendication 1, dans lequel lesdites fibres optiques (309) sont alignées.

3. Élément capteur selon la revendication 1 ou 2, dans lequel ledit élément de fenêtre comprend une platine de fibre optique.

4. Élément capteur selon la revendication 1 ou 2, dans lequel ledit élément de fenêtre comprend un effilement de fibre optique.

5. Élément capteur selon l'une quelconque des revendications précédentes, dans lequel les fibres optiques (309, 409, 419, 429) de l'élément de fenêtre (307, 707) sont configurées pour guider la lumière qui provient de la source de lumière (202) vers l'extérieur de l'élément capteur ou de l'extérieur de l'élément capteur vers le détecteur de lumière (203) .

6. Élément capteur selon l'une quelconque des revendications précédentes, dans lequel l'élément de fenêtre comprend un support (401, 411, 412, 421) entre les fibres optiques.

7. Élément capteur selon la revendication 6, dans lequel le support entre les fibres optiques comprend un matériau de revêtement qui entoure le matériau central des fibres optiques.

8. Élément capteur selon la revendication 6 ou 7, dans lequel le support entre les fibres optiques est un matériau teinté.

9. Élément capteur selon l'une quelconque des revendications 5 à 8, dans lequel le support entre les fibres optiques est non-transparent sur une certaine plage de longueurs d'onde.

10. Élément capteur selon l'une quelconque des revendications 6 à 9, dans lequel le support entre les fibres optiques est non-transparent à la lumière visible.

11. Élément capteur selon l'une quelconque des revendications 6 à 10, dans lequel le support entre les fibres optiques et le matériau central des fibres optiques sont teintés.

12. Appareil qui comprend un capot et un élément capteur selon l'une quelconque des revendications 6 à 11, dans lequel au moins une partie du capot et le support entre les fibres optiques de l'élément de fenêtre de l'élément capteur possèdent la même couleur afin de camoufler le contenu de l'élément capteur.

13. Procédé qui comprend :
le fait de prévoir (1101) un élément capteur qui comprend une source de lumière (202) configurée pour émettre de la lumière vers l'extérieur de l'élément capteur et un détecteur de lumière (203) configuré pour détecter la lumière qui pénètre dans l'élément capteur, dans lequel l'élément capteur est un capteur optique configuré pour détecter des conditions physiologiques, **caractérisé par**
le fait de prévoir des parois (204-206) destinées à entourer la source de lumière (202) et le détecteur de lumière (203), et l'utilisation (1102) d'un élément de fenêtre (307, 707, 808, 809, 907) en contact avec les parois afin de recouvrir et de protéger la source de lumière (202) et le détecteur de lumière (203), dans lequel l'élément de fenêtre comprend une pluralité de fibres optiques adjacentes (309, 409, 419, 429).

14. Procédé selon la revendication 13, dans lequel l'élément de fenêtre comprend un support teinté entre les fibres optiques (309, 409, 419, 429).
